# EUROPÄISCHE PATENTSCHRIFT

(11) **EP 1 039 298 B1**
(45) Veröffentlichungstag und Bekanntmachung des Hinweises auf die Patenterteilung: **04.08.2004**
(21) Anmeldenummer: 00105264.6
(22) Anmeldetag: 14.03.2000
(51) Int. Cl.: G01N 33/52, C09J 4/06

(54) **Mehrschichtiges analytisches Testelement**
Multilayer analytical test element
Elément d'essai à plusieurs couches

(30) Priorität: 19.03.1999 DE 19912365
(43) Veröffentlichungstag der Anmeldung: 27.09.2000
(73) Patentinhaber: Roche Diagnostics GmbH, 68298 Mannheim (DE)
(72) Erfinder: Zimmer, Volker, 69221 Dossenheim (DE); Braun, Hans-Peter, Dr., 69469 Weinheim (DE)

(56) Entgegenhaltungen:
- WO-A-97/27483
- DE-U- 29 509 296
- GB-A- 1 512 352
- US-A- 4 301 115
- US-A- 5 248 708

## Beschreibung

Die Erfindung betrifft ein analytisches Testelement, enthaltend mindestens zwei Komponenten, wobei zumindest zwei der Komponenten mit Hilfe eines Klebstoffs miteinander verbunden sind.

Zur schnellen und einfachen qualitativen oder quantitativen analytischen Bestimmung von Bestandteilen von flüssigen Proben, z. B. wäßrigen Körperflüssigkeiten wie Blut, Serum oder Urin, werden oft sogenannte trägergebundene Tests (Testträger, Testelemente, Teststreifen) verwendet. Bei diesen trägergebundenen Tests sind Nachweisreagenzien in entsprechenden Schichten eines Trägers eingebettet, der mit der flüssigen Probe in Kontakt gebracht wird. Die Reaktion von flüssiger Probe und Reagenzien führt bei Anwesenheit eines Zielanalyten zu einem nachweisbaren Signal, z. B. einem meßbaren elektrischen Signal oder einem Farbumschlag, welcher visuell oder mit Hilfe eines Geräts, z. B. reflexionsphotometrisch, ausgewertet werden kann.

Analytische Testelemente oder Testträger sind häufig als Teststreifen ausgebildet, die im wesentlichen aus einem länglichen Trägermaterial aus Kunststoff und darauf angebrachten Nachweisschichten als Testfeldern bestehen. Die Montage der Testelemente wird in vielen Fällen durch Verschweißen oder Kleben von Trägermaterial und Nachweisschicht durchgeführt. Insbesondere das Verschweißen mittels Ultraschall und das Kleben mit Schmelzklebstoffen (sog. "Hotmelts"), härtenden Kaltklebstoffen oder Klebebändern hat sich für die Fertigung von Testelementen in hohen Stückzahlen als vorteilhaft erwiesen, wobei wegen der möglichen hohen Produktionsgeschwindigkeit die Fertigung mit Hilfe von Klebebändern zunehmend häufiger Verwendung findet. Testelemente, die unter Verwendung von Klebstoffen gefertigt werden, sind beispielsweise in den Deutschen Patentanmeldungen mit Aktenzeichen P 197 53 847.9 und P 198 15 684.7, in der deutschen Gebrauchsmusteranmeldung U 29509296, sowie in EP-A 0 212 314, EP-A-0 297 390, EP-A-0 297 389, EP-A 0 821 234 und EP-A 0 821 233 beschrieben.

Aus WO 97/27483 sind Teststreifen bekannt, die ein Trockenmittelreservoir in Form eines in Schmelzklebstoff eingebetteten Trockenmittels enthalten.

Obwohl die Fertigung von analytischen Testelementen mit Hilfe von Klebstoffen weit verbreitet ist, treten bei Testelementen verschiedentlich Probleme auf, die im Zusammenhang mit der Verwendung von Klebstoffen stehen. Insbesondere ist es bekannt, daß Klebstoffe nachteilige Auswirkungen auf die Stabilität der Testelemente haben können. Hier wird vor allem angenommen, daß die Reagenzien in der Nachweisschicht durch Inhaltsstoffe der Klebstoffe, die zum Verbinden der einzelnen Testelementkomponenten verwendet werden, negativ in ihrer Stabilität beeinflußt werden. Deshalb versucht man bei der Optimierung der Testelementstabilität unter anderem, durch geeignete Wahl der verwendeten Klebstoffe Stabilitätsprobleme zu minimieren. Aufgrund der sehr unterschiedlichen, zum Teil sehr empfindlichen Reagenzien in Nachweisschichten und der großen Vielfalt von prinzipiell für die Testelementefertigung verfügbaren Klebstoffe kann die optimale Klebstoffwahl zeit- und kostenintensiv sein.

Analytische Testelemente für wäßrige Probenflüssigkeiten, wie z. B. Körperflüssigkeiten, werden in denjenigen Bereichen, an denen sie vom Probenmaterial benetzt werden sollen, beispielsweise also im Bereich der Nachweisschicht, oftmals chemisch und/oder physikalisch behandelt, um das Benetzen zu erleichtern. Beispielsweise ist aus der Deutschen Patentanmeldung Aktenzeichen P 197 53 848.7 bekannt, daß an sich hydrophobe, d. h. wasserabweisende Flächen durch Behandlung mit Netzmitteln oder durch Beschichtung mit oxidiertem Aluminium hydrophil gemacht werden können und so ein Benetzen der Flächen mit wäßrigen Probenflüssigkeiten erleichtern oder erst ermöglichen. Derartig behandelte Flächen in Testelementen zeigen in Gegenwart von Klebstoffen ebenfalls Stabilitätsprobleme, die sich durch schlechte Benetzbarkeit mit wäßrigen Flüssigkeiten bemerkbar macht. Dieses Problem tritt nicht nur bei Testelementen auf, sondern ist auch bei anderen analytischen Hilfsmitteln, bei denen die Benetzung von Oberflächen eine Rolle spielt, z. B. Probennahmeelementen, Küvetten oder ähnlichem, von Interesse. Dies gilt insbesondere, wenn die Flüssigkeitsaufnahme in das analytische Hilfsmittel mit Hilfe von Kapillarkräften geschieht.

Aufgabe der Erfindung ist es, die Nachteile des Standes der Technik zu beseitigen. Insbesondere ist es die Aufgabe der vorliegenden Erfindung, die Stabilität von analytischen Hilfsmitteln, insbesondere Testelementen, bei denen Klebstoffe zur Montage der einzelnen Komponenten verwendet werden, zu erhöhen.

Diese Aufgabe wird durch den Gegenstand der Erfindung, wie er in den Patentansprüchen charakterisiert ist, gelöst.

Gegenstand der Erfindung ist ein analytisches Hilfsmittel wie es in Anspruch 1 definiert ist.

Die erfindungsgemäßen analytischen Hilfsmittel sind vorzugsweise zur Verwendung mit Probenflüssigkeiten geeignet. Es ist jedoch auch möglich, feste oder gasförmige Stoffe mit ihnen zu untersuchen, sofern diese Stoffe vor oder während der Analyse mit einem flüssigen Reaktionsmedium, beispielsweise einem Puffer, in Kontakt gebracht und darin gelöst oder suspendiert werden. Als Probenflüssigkeiten kommen insbesondere wäßrige Flüssigkeiten, z. B. biologische Proben wie Blut, Urin, Schweiß oder Speichel, oder wäßrige Lösungen oder Suspensionen von Gasen oder Feststoffen in Frage.

Unter dem Begriff "analytische Hilfsmittel" sollen analytische Testelemente verstanden werden.

Vorzugsweise können analytische Hilfsmittel analytische Testelemente sein, bei denen bereits während oder nach der Aufnahme der Probenflüssigkeit geeignete Nachweisreaktionen ablaufen, welche die Bestimmung der Anwesenheit oder Menge eines Analyts in der Probe erlauben. Analytische Testelemente im hier benutzten Sinn sind visuell oder apparativ-optisch auswertbare Testelemente, z.B. Teststreifen, Biosensoren, wie z.B. enzymatische Biosensoren oder optische Biosensoren (Optroden, Wellenleiter etc.), elektrochemische Sensoren und dergleichen mehr. Vorzugsweise werden in den analytischen Testelementen enzymatische, immunologische oder auf Nukleinsäuren basierende Methoden zum Analytnachweis eingesetzt. Derartige analytische Hilfsmittel sind im Stand der Technik umfassend beschrieben und dem Fachmann in einer Vielzahl von Ausführungsformen geläufig. Beispielsweise sei auf folgende Dokumente verwiesen: Deutsche Patentanmeldung Aktenzeichen 197 53 847.9, EP-A 0 138 152, EP-A 0 821 234; EP-A 0 821 233, EP-A 0 750 185, EP-A 0 650 054, EP-A-0 471 986, EP-A 0 699 906 und WO 97/02487.

Erfindungsgemäß enthält das analytische Hilfsmittel vorzugsweise mindestens zwei Komponenten. Vorzugsweise enthält das erfindungsgemäße analytische Hilfsmittel einen Träger, der bevorzugt schichtförmig ist und der mit mindestens einem weiteren Material mit Hilfe eines Klebstoffs verbunden ist. Im Falle eines analytischen Testelements kann das zweite Material beispielsweise eine Nachweisschicht, eine Abtrennschicht, bspw. für partikuläre Probenbestandteile, die nicht in die Nachweisschicht vordringen sollen, eine Flüssigkeitstransportschicht, eine Spreitschicht oder eine Schicht zur Aufnahme überschüssiger Probe sein. Es ist auch möglich, daß im Falle von mehr als zwei Komponenten nicht der Träger mit Hilfe des Klebstoffs mit einer anderen Komponente verbunden ist, sondern daß zwei der übrigen Komponenten, beispielsweise Abtrennschicht und Nachweisschicht, mit einem Klebstoff verbunden sind. Als schichtförmig im erfindungsgemäßen Sinn sollen dabei nicht nur ebene Schichten, sondern auch gekrümmte, bspw. gewellte Schichten verstanden werden.

Als Träger kommen für das erfindungsgemäße analytische Hilfsmittel eine Reihe von Materialien in Frage, die üblicherweise zur Fertigung von analytischen Hilfsmitteln, beispielsweise Testelementen, eingesetzt werden, wie z. B. Metall- oder Kunstoffolien, beschichtete Papiere oder Pappen, sowie - wenn auch weniger bevorzugt - Glas. Bevorzugt ist der Träger aus einer opaken oder transparenten Kunststoffolie gefertigt, beispielsweise aus Polyethylen, Polypropylen, Polyethylenterephthalat, Polystyrol oder Polycarbonat. Für die übrigen, oben erwähnten Komponenten sind erfindungsgemäß ebenfalls die dem Fachmann aus dem Stand der Technik bekannten Materialien geeignet: als Nachweisschichten eignen sich reagenzienimprägnierte Vliese (vgl. bspw. DE-A 196 22 503), Papiere (vgl. bspw. DE-A 27 16 060), Membranen (vgl. bspw. US 5,451,504) oder als Film auf eine Tragschicht aufgebrachte Beschichtungsmassen (vgl. bspw. DE-A 196 29 656); als Abtrennschichten sind Membranen (vgl. bspw. DE-A 39 22 495) oder Vliese (vgl. bspw. EP-A 0 045 476) geeignet; als Transportschichten für die Probenflüssigkeit oder als Schichten zur Aufnahme überschüssiger Probe eignen sich chromatographiefähige Vliese (vgl. bspw. EP-A 0 339 450), Papiere (vgl. bspw. US 4,861,711), Gewebe (vgl. bspw. DE-A 196 29 657) oder Membranen (vgl. bspw. US 5,451,504); als Spreitschichten eignen sich u. a. Gewebe, die gegebenenfalls mit Hilfsstoffen imprägniert sind (vgl. bspw. DE-A 196 29 657).

Zur Verbindung zweier der mindestens zwei Komponenten des erfindungsgemäßen analytischen Hilfsmittels wird ein Klebstoff eingesetzt. Je nachdem, welche Art von Verbindung gewünscht wird oder welche Montagetechnik angewandt wird, ist es möglich, daß der Klebstoff in Form eines Klebstoffilms, einer Klebstoffschicht, einzelner Klebstoffbezirke, z. B. in Form eines Punkt- oder Linienmusters, oder als einseitig oder beidseitig klebendes Klebeband eingesetzt wird. Ebenso kann je nach Art der zu verbindenden Schichten der Klebstoff aus der Gruppe der physikalisch abbindenden Klebstoffe, zu der u. a. Leime, Kleister, Lösungsmittelklebstoffe, Dispersionsklebstoffe und Schmelzklebstoffe gehören, oder der chemisch abbindenden Klebstoffe ausgewählt werden. Vorzugsweise werden für die erfindungsgemäßen analytischen Hilfsmittel physikalisch abbindende, lösungsmittelhaltige oder lösungsmittelfreie Klebstoffe verwendet. Besonders bevorzugt sind lösungsmittelfreie, physikalisch abbindende Klebstoffe, wobei diese in Form von Klebebändern, insbesondere beidseitig klebenden Klebebändern ganz besonders bevorzugt sind. Es hat sich herausgestellt, daß erfindungsgemäß insbesondere Klebebänder enthaltend Acrylatklebstoff hervorragende Ergebnisse zeigt.

Das erfindungsgemäße analytische Hilfsmittel zeichnet sich dadurch aus, daß im Klebstoff, der zum Verbinden zweier der mindestens zwei Komponenten eingesetzt wird, ein adsorptionsfähiges Material enthalten ist. Klebstoffe, die prinzipiell adsorptionsfähiges Material wie Ruß oder Aktivkohle enthalten, sind im Stand der Technik beschrieben, ohne daß jedoch die hier beschriebene, erfindungsgemäße Verwendung zur Stabilisierung von analytischen Hilfsmiteln angesprochen oder nahegelgt wird. In JP 56010458 und JP 56010459 wird Aktivkohle zwischen zwei schichtförmige, z. T. gasdurchlässige Materialien mit Hilfe einer Schmelzkleberschicht eingebracht und dieses Laminat als Verpackungsmaterial für Lebensmittel verwendet, um so aus der Luft Spurenstoffe zu adsorbieren, die zum Verderben von Obst und Gemüse führen. Ruß in Klebstoffen ist u. a. aus DE-A 1594226 zur Stabilisierung lichtempfindlicher Klebstoffe, aus JP 2011684 zur Erzeugung druckempfindlicher Klebstoffe, aus Zhongguo Jiaonianji **4** (1995) 31-33 zur Herstellung antistatischer Klebstoffe sowie aus EP-A 0 174 188 als mikrowellensensitiver Zusatz zu Klebstoffen bekannt.

Überraschenderweise hat sich gezeigt, daß die Gegenwart eines adsorptionsfähigen Materials im Klebstoff zu einer deutlichen Steigerung der Stabilität der untersuchten analytischen Hilfsmittel führt. Während mit herkömmlichen, keine adsorptionsfähigen Materialien enthaltenden Klebstoffen hergestellte analytische Hilfsmittel mit der Zeit deutliche Verschlechterungen bestimmter Eigenschaften, wie Benetzbarkeit der mit Probenflüssigkeit in Kontakt kommenden Bereiche oder Unversehrtheit von Reagenzien, zeigen, können diese negativen Auswirkungen durch den Zusatz von adsorptionsfähigen Materialien zum Klebstoff vermindert oder gar verhindert werden. Man nimmt an, daß dieser stabilisierende Effekt der Adsorptionsmittel im Klebstoff darauf zurückzuführen ist, daß flüchtige oder migrationsfähige, die Stabilität des analytischen Hilfsmittels beeinträchtigende Substanzen, die im Klebstoff enthalten sind, durch das Adsorptionsmittel im Klebstoff gehalten werden und entweder nur noch mehr oder weniger stark verzögert oder überhaupt nicht mehr aus dem Klebstoff auf empfindliche Bereiche des analytischen Hilfsmittels übertreten können. Dies ist umso überraschender, als das Adsorptionsmittel allseitig von Klebstoff umgeben ist, dennoch aber seine Adsorptionsfähigkeit behält.

Insbesondere bei analytischen Hilfsmitteln, die eine kapillaraktive Zone, beispielsweise einen Kapillarspalt oder ein kapillaraktives Vlies, Papier oder Gewebe enthalten, hat sich der Zusatz eines adsorptionsfähigen Stoffes als vorteilhaft herausgestellt. Das Vorhandensein einer kapillaraktiven Zone, insbesondere eines Kapillarspaltes, ermöglicht in den erfindungsgemäßen analytischen Hilfsmitteln ein selbständiges Aufnehmen eines - bei entsprechend genauer und reproduzierbarer Fertigung der kapillaraktiven Zone - definierten Probenvolumens und ist daher besonders bevorzugt. Die kapillaraktive Zone kann dabei beliebig geformt sein, solange zumindest in einer Dimension Kapillarität gewährleistet ist. Insbesondere kann das erfindungsgemäße analytische Hilfsmittel eine Kapillare enthalten, die aus einer steifen Trägerfolie und einer gegebenenfalls identischen Abdeckfolie besteht, welche durch eine Distanzhalterschicht, in die eine Aussparung in Form der Kapillare eingebracht ist, derartig verbunden sind, daß zwischen ihnen ein Kapillarspalt entsteht. Vorzugsweise wird als Distanzhalterschicht ein beidseitig klebendes Klebeband eingesetzt, bei welchem dem Klebstoff ein adsorptionsfähiges Material zugesetzt wurde.

Während sich das Füllverhalten von Kapillaren in analytischen Hilfsmitteln, die mit herkömmlichen, keine Adsorptionsmittel enthaltenden Klebstoffen hergestellt werden, mit der Zeit verschlechtert, wird durch die Verwendung von Klebstoffen. die als Zusatz ein Adsorptionsmittel enthalten, die Zeit, die benötigt wird, um die Kapillare im analytische Hilfsmittel mit Probenflüssigkeit zu füllen, auch bei längerer Lagerung und bei höheren Temperaturen im wesentlichen konstant gehalten. Dies trifft vor allem bei wäßrigen Probenflüssigkeiten zu, die mit analytischen Hilfsmitteln, bei welchen die kapillaraktive Zone im wesentlichen aus an sich unpolaren Materialien gefertigt sind und die deshalb mit hydrophilierenden Mitteln behandelt sind, untersucht werden. Offensichtlich führen gewisse Inhaltsstoffe von Klebstoffen dazu, daß die hydrophilierende, die Benetzbarkeit unpolarer Oberflächen steigernde Wirkung aufgehoben oder verringert wird. Werden Adsorptionsmittel zum Klebstoff zugesetzt, wie dies erfindungsgemäß beschrieben ist, kann der negative Einfluß besagter Inhaltsstoffe vermindert oder sogar aufgehoben werden. Als Folge davon bleiben die Oberflächen des erfindungsgemäßen analytischen Hilfsmittels weitgehend unverändert, was sich bei Kapillaren dadurch äußert, daß die Füllzeit für eine derartig hergestellte Kapillare konstant bleibt.

Als adsorptionsfähige Materialien, oder Adsorbentien oder Adsorptionsmittel, haben sich erfindungsgemäß insbesondere feste Stoffe, die aufgrund ihrer großen Oberfläche befähigt sind, Stoffe aus gasförmigen oder flüssigen Mischungen an ihrer Grenzfläche durch Physiund/oder Chemisorption selektiv anzureichern, als geeignet herausgestellt. Dabei bestimmt die Wahl des Adsorbens, welche Stoffe bevorzugt adsorbiert werden können. Je feiner eine bestimmte Menge des Adsorbens zerteilt ist, um so höher ist auch ihre Adsorptionsfähigkeit. Insbesondere sind poröse Feststoffe mit narbigen Oberflächen erfindungsgemäß bevorzugt, z. B. Aktivkohlen, Aluminiumoxide, Kieselgele, Ruße oder Zeolithe, wobei es besonders bevorzugt ist, daß diese Feststoffe nicht im Klebstoff löslich sind. Ganz besonders bevorzugt ist Ruß oder Aktivkohle als Adsorptionsmittel. Vorzugsweise wird dem Klebstoff nur eine Sorte Adsorptionsmittel zugemischt. Es ist jedoch auch möglich, beliebige, durch Routineversuche optimierbare Mischungen von Adsorptionsmitteln mit dem Klebstoff zu vermischen.

Um die Klebeeigenschaften und die Verarbeitbarkeit des Klebstoffs durch den Zusatz adsorptionsfähiger Materialien nicht unnötig zu beeinflussen hat es sich als vorteilhaft herausgestellt, dem Klebstoff nicht mehr als 40 Gew.-% Adsorbens, bezogen auf die getrocknete Gesamtmasse des Klebstoffs, beizumischen. Andererseits tritt die vorteilhafte Wirkung des Adsorptionsmittels unterhalb von 1 Gew.-% nicht ein. Vorzugsweise werden dem Klebstoff 1 bis 30 Gew.-% Adsorbens, besonders bevorzugt 5 bis 30 Gew.-% zugesetzt.

Die optimale Menge an Adsorptionsmittel hängt naturgemäß von dessen Art, dessen inneren und äußeren Oberfläche, dessen Partikelgröße und Feinverteilung ab, wobei als Kriterien einerseits die Klebekraft und Verabeitbarkeit der resultierenden Klebstoffmasse und andererseits der gewünschte stabilisierende Effekt des Adsobenszusatzes zu berücksichtigen sind. Optimale Gewichtsanteile können vom Fachmann anhand einfacher Versuche ermittelt werden.

Die erfindungsgemäßen analytischen Hilfsmittel weisen die folgenden Vorteile auf:
◆ Schädigende Einflüsse von Klebstoffbestandteilen auf die Reagenzien in erfindungsgemäßen analytischen Hilfsmitteln werden vermindert. Die Lagerstabilität der erfindungsgemäßen analytischen Hilfsmittel wird dadurch erhöht.
◆ Negative Auswirkungen auf hydrophilierte Bezirke in analytischen Hilfsmitteln werden minimiert. Dadurch wird die Benetzbarkeit dieser Bezirke durch wäßrige Probenflüssigkeiten stabilisiert.

Die Erfindung wird durch die nachfolgenden Beispiele und die Zeichnung näher erläutert.

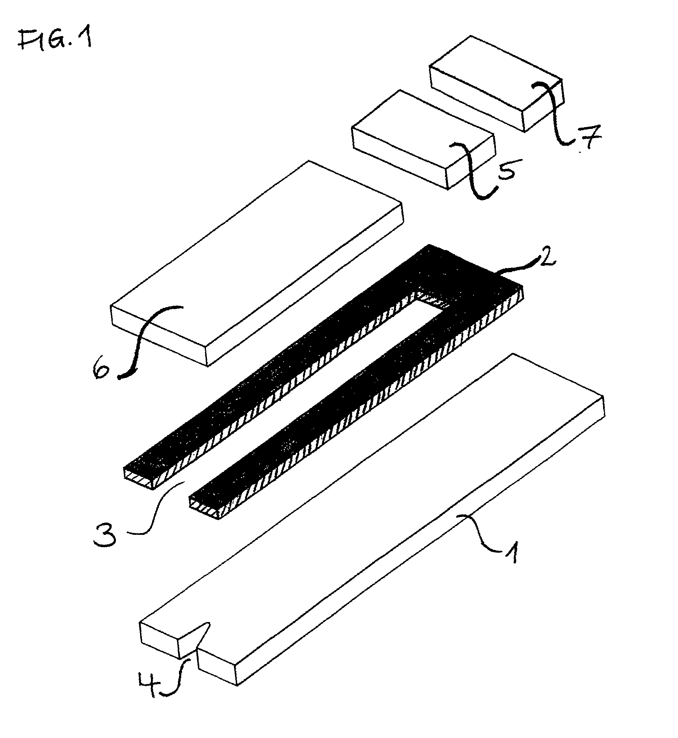

Figur 1 zeigt schematisch anhand einer Explosionszeichnung eine besonders bevorzugte Ausführungsform eines erfindungsgemäßen analytischen Testelements.

Die Ziffern in der Figur bedeuten:
1 Trägerschicht
2 Distanzhalterschicht (Spacer)
3 kapillaraktiver Kanal
4 Aussparung in der Trägerschicht
5 Nachweisfilm
6 Abdeckfolie
7 Schutzfolie

In Figur 1 ist eine bevorzugte Ausführungsform eines erfindungsgemäßen analytischen Testelements schematisch in einer Explosionszeichnung abgebildet. Auf einer Trägerschicht 1, in die eine Aussparung 4 in Form einer V-förmigen Kerbe eingebracht wurde, die unter anderem der Markierung der Probenaufgabestelle dient, befindet sich eine Distanzhalterschicht 2, welche die Kontur und die Höhe (entsprechend der Dicke der Distanzhalterschicht 2) eines kapillaraktiven Kanals 3 bestimmt. Die Distanzhalterschicht 2 besteht aus einem beidseitig klebenden Klebeband, dem Aktivkohle zur Klebstoffmasse beigemischt wurde. Auf dieser Distanzhalterschicht 2 kommen eine Abdeckfolie 6, ein Nachweisfilm 5, sowie eine Schutzfolie 7 zu liegen. Abdeckfolie 6 und Nachweisfilm 5 werden so eng nebeneinander montiert, daß die kapillaraktive Zone 3 ununterbrochen von der freien, über der Kerbe 4 liegenden Kante der Abdeckfolie 6 bis zur gegenüberliegenden, freien Kante des Nachweisfilms 5 reicht. Die Aussparung in der Distanzhalterschicht 2, welche die Form des kapillaraktiven Kanals 3 bestimmt, ist geringfügig länger gehalten als Abdeckfolie 6 und Nachweiselement 5 zusammen, so daß ein in aller Regel wenige Millimeter breiter, unbedeckter Spalt verbleibt, aus dem beim Befüllen der kapillaraktiven Zone 3 mit Probenflüssigkeit Luft entweichen kann. Dieser Spalt bleibt auch durch die Schutzfolie 7 unbedeckt, damit seine Funktion gewahrleistet bleibt. Die Schutzfolie 7 soll verhindern, daß frei liegende Bereiche des Klebebandes 2 zu unerwünschtem Verkleben des Testelements mit Gegenständen aus der Umgebung führen.

### Beispiel 1

### Herstellung eines erfindungsgemäßen analytischen Testelements gemäß Figur 1

Auf eine mit einer 30 nm dicken Schicht aus Aluminium, das analog zur deutschen Patentanmeldung mit dem Aktenzeichen P 197 53 848.7 mit Wasserdampf vollständig oxidiert wurde, beschichtete, 350 µm dicke Folie aus Polyethylenterephthalat (Melinex®, ICI, Frankfurt am Main, Deutschland) wurde ein doppelseitiges Klebeband der Dicke 100 µm geklebt, welches aus einer 50 µm dicken Polyethylenterephthalatfolie (Melinex®, ICI, Frankfurt am Main, Deutschland) bestand, die beidseitig mit je einer 25 µm dicken Schicht aus Klebstoff (Duro-Tak 373-0102, National Starch), dem bezogen auf das Gesamtgewicht 30 Gew.-% Aktivkohle (Pulsorb GW, Partikelgröße kleiner als 18 µm, Chemviron Carbon) zugesetzt war, beschichtet war. Die Folie hatte eine Länge von 25 mm und war 5 mm breit. An einer der kurzen Seiten befand sich eine zentrale, kerbenförmige Aussparung von 1 mm Breite und 2 mm Länge. Das Klebeband besaß eine Ausstanzung von 2 mm Breite und mehr als 15 mm Länge, welche die Dimensionen des Kapillarkanals definiert. Die Länge der Ausstanzung war geringfügig größer als die gewünschte Länge des kapillaraktiven Kanals, die durch dessen Abdeckung bestimmt wurde, um eine Entlüftung des Kanals während des Befüllens mit Probenflüssigkeit zu gewährleisten. Auf das Klebeband wurde auf der Seite, an der die Entlüftung vorgesehen war, in 1 mm Abstand vom Ende der Ausstanzung ein 3 mm langer und 5 mm breiter Nachweisfilm geklebt. Als Nachweisfilm wurde ein Film verwendet, wie er aus DE-A 196 29 656 bekannt ist. Der Nachweisfilm war spezifisch für den Nachweis von Glucose. Auf den noch offen liegenden Bereich des Klebebandes zwischen kerbenförmiger Aussparung und Nachweisfilm wurde eine 12 mm lange und 5 mm breite Abdeckschicht aufgeklebt, so daß Abdeckschicht und Nachweisfilm Stoß an Stoß zu liegen kamen. Die Abdeckschicht bestand aus einer 150 µm dicken, einseitig mit Klebstoff versehenen Polyethylenterephthalat-Folie, auf die auf der zum Kapillarkanal hingewandten Seite eine mit, wie oben beschreiben, oxidiertem Aluminium der Dicke 30 nm beschichtete, 6 µm dicke Polyethylenterephthalat-Folie geklebt war ( beide: Hostaphan®, Hoechst, Frankfurt am Main, Deutschland). Um noch frei liegende Klebebandbereiche abzudecken wurden diese mit einer 175 µm dicken Folie aus Polyethylenterephthalat (Melinex®, ICI, Frankfurt am Main, Deutschland) abgedeckt, ohne dabei jedoch funktionale Bereiche abzudecken.

Das so erhaltene Testelement hatte einen kapillaren Kanal von 15 mm Länge, 2 mm Breite und 0,1 mm Höhe. Der Kanal konnte 3 µl Probenflüssigkeit aufnehmen. Der Nachweisfilm wurde auf einer Fläche von 3 mm × 2 mm von der Probe benetzt.

### Beispiel 2

### Zeitabhängiges Füllverhalten von Kapillaren

Acrylatklebstoff (Duro-Tak 373-0102, National Starch) wurde mit Zusatz von 30 Gew.-% Aktivkohle (Pulsorb GW, Partikelgröße kleiner als 18 µm, Chemviron Carbon) bezogen auf die Gesamtmasse des Klebstoffs in zwei identischen, jeweils 25 µm dicken Schichten auf eine 50 µm dicke Trägerfolie aus Polyethylenterephthalat (Melinex®, ICI, Frankfurt am Main, Deutschland) aufgebracht und so zu einem beidseitig klebenden Klebeband verarbeitet. Aus einem 5 × 20 mm² großen Stück des so hergestellten Klebebandes wurde ein rechteckiges Stück der Größe 2 × 16 mm² herausgeschnitten, so daß ein symmetrischer, U-förmiger Klebebandrest verblieb. Dieser wurde bündig auf eine 5 × 20 mm² große, 350 µm dicke, unbehandelte Polyethylenterephthalatfolie (Melinex®, ICI, Frankfurt am Main, Deutschland) geklebt und auf der verbleibenden Oberfläche mit einer 5 × 15 mm² großen, 350 µm dicken Folie aus Polyethylenterephthalat (Melinex® , ICI, Frankfurt am Main, Deutschland), die zuvor auf der dem Klebeband zugewandten Seite mit einer 30 nm dicken Schicht aus Aluminium, das, analog zu Beispiel 1, mit Wasserdampf vollständig oxidiert wurde, beschichtet worden war, abgedeckt. Die kleinere der beiden Polyethylenterephthalatfolien bedeckte dabei die beiden parallelen Schenkel des U-förmigen Klebebandes und schloß bündig mit der Öffnung des U ab. Auf diese Weise wurde ein rechteckiger, kapillarer Hohlraum mit den Dimensionen 2 × 15 × 0,1 mm³ erzeugt, der auf einer Seite offen war, um dort Flüssigkeit aufzunehmen. Analog hierzu wurde eine Kapillare gefertigt, bei der das Klebeband keinen Aktivkohlezusatz enthielt.

Mit den so hergestellten Kapillaren mit aktivkohlehaltigen und aktivkohlefreien Klebebändern wurden mit EDTA-Venenblut (Hämatokrit 42 %) Füllversuche durchgeführt. Dabei wurde die Zeit bestimmt, die von der Probenflüssigkeit benötigt wurde, um den kapillaren Hohlraum vollständig zu füllen. Die Ergebnisse sind in Tabelle 1 zusammengefaßt.

**Tabelle 1**

| **Zeitpunkt der Messung** | **Füllzeit bei Verwendung von Klebeband ohne Aktivkohiezusatz** | **Füllzeit bei Verwendung von Klebeband mit Aktivkohlezusatz** |
|---|---|---|
| Sofort nach Fertigung der Kapillare | 2,5 s | 2,5 s |
| Nach 4 Wochen Lagerung bei Raumtemperatur (21 -23 °C) | 3,8 s | 2,5 s |
| Nach 4 Wochen Lagerung bei 35°C | > 10 s | 2,6 s |

Wie aus Tabelle 1 zu erkennen ist, wurde mit der erfindungsgemäßen Verwendung eines aktivkohlehaltigen Klebebandes sowohl bei Raumtemperatur als auch bei erhöhter Lagertemperatur eine deutliche Steigerung der Stabilität der Kapillare erzielt.

### Beispiel 3

### Zeitabhängiges Füllverhalten von Kapillaren enthaltend unterschiedliche adsorptionsfähige Materialien

Analog zu Beispiel 2 wurden Kapillaren gefertigt. Dem Acrylatklebstoff (Duro-Tak 373-0102, National Starch) waren in diesem Fall die folgenden, getrockneten, unterschiedlichen adsorptionsfähigen Materialien beigefügt:
1.) Aktivkohle (Pulsorb GW, Chemviron Carbon)
2.) Silicagel
3.) Aluminiumoxid (G 60 neutral, Merck)
4.) Molekularsieb

In allen Fällen betrug der Anteil an adsorptionsfähigem Material nach dem Trocknen des Klebstoffs 15 Gew.-%.

Die so erhaltenen Kapillaren wurden 3,5 Wochen jeweils bei Raumtemperatur (RT), bei 35°C und bei 50°C eingelagert. Die analog Beispiel 2 ermittelten Füllzeiten der 3,5 Wochen alten Kapillaren wurden mit den Füllzeiten einer frisch hergestellten Kapillare verglichen, bei der lediglich die aluminiumoxidbeschichtete Folie, nicht jedoch dia übrigen Komponenten, bei den oben genannten Temperaturen gelagert wurden. Zum Vergleich wurde eine Kapillare herangezogen, die keinen Zusatz eines adsorptionsfähigen Materials im Klebstoff enthielt und ebenfalls für 3,5 Wochen bei den oben genannten Temperaturen eingelagert war. Die Mittelwerte der Füllzeiten aus jeweils 8 Messungen für jede der untersuchten Kapillaren sind in Tabelle 2 wiedergegeben.

**Tabelle 2**

| **Lagertemperatur** | **Füllzeit (in s) der Kapillaren mit unterschiedlichen Zusätzen nach 3,5 Wochen Lagerung (Mittelwerte aus n=8 Messungen)** | | | | | **frisch hergestellte Vergleichskapillare** |
|---|---|---|---|---|---|---|
| | **kein Zusatz** | **Aktivkohle** | **Silicagel** | **Aluminium oxid** | **Molekularsieb** | |
| RT | >10 | 2,4 | 2,6 | 3,2 | 3,4 | 2,4 |
| 35°C | >10 | 3,2 | 2,1 | 3.9 | 3,9 | 2,4 |
| 50°C | >10 | 3,5 | 2,5 | 6,6 | >10 | 2,5 |

## Patentansprüche

1. Analytisches Testelement zur Untersuchung einer flüssigen Probe enthaltend einen Träger und eine Komponente,
wobei die eine Komponente ausgewählt ist aus der Gruppe
umfassend eine Nachweisschicht, eine Abtrennschicht, eine
Flüssigkeitstransportschicht, eine Spreitschicht und eine Schicht zur Aufnahme überschüssiger Probe,
wobei der Träger mit der einen Komponenten mit Hilfe eines Klebstoffs miteinander verbunden ist, und/oder die eine Komponente mit zumindest einer weiteren Komponente, ausgewählt aus der Gruppe umfassend eine Nachweisschicht, eine Abtrennschicht, eine Flüssigkeitstransportschicht, eine Spreitschicht und eine Schicht zur Aufnahme überschüssiger Probe, mit Hilfe des Klebstoffs miteinander verbunden ist
**dadurch gekennzeichnet, daß**
der Klebstoff einen porösen Feststoff als adsorptionsfähiges Material enthält,
wobei der poröse Feststoff ausgewählt ist aus der Gruppe umfassend Aktivkohle, Ruß, Aluminiumoxid, Kieselgel oder Zeolith.

2. Analytisches Testelement gemäß Anspruch 1, **dadurch gekennzeichnet, daß** der Feststoff nicht im Klebstoff löslich ist.

3. Analytisches Testelement gemäß Anspruch 1 oder 2, **dadurch gekennzeichnet, daß** der Feststoff Aktivkohle oder Ruß ist.

4. Analytisches Testelement gemäß einem der Ansprüche 1 bis 3, **dadurch gekennzeichnet, daß** der Anteil des adsorptionsfähigen Materials 1 bis 40 Gew.-% bezogen auf die getrocknete Gesamtmasse des Klebstoffs beträgt.

5. Analytisches Testelement gemäß einem der Ansprüche 1 bis 4, **dadurch gekennzeichnet, daß** der Klebstoff physikalisch abbindend ist.

6. Analytisches Testelement gemäß Anspruch 5, **dadurch gekennzeichnet, daß** der Klebstoff lösungsmittelfrei ist.

7. Analytisches Testelement gemäß Anspruch 6, **dadurch gekennzeichnet, daß** der Klebstoff ein Acrylatklebstoff ist.

8. Analytisches Testelement gemäß einem der Ansprüche 5-7, **dadurch gekennzeichnet, daß** der Klebstoff in Form eines Klebebandes vorliegt.

9. Analytisches Testelement gemäß Anspruch 8, **dadurch gekennzeichnet, daß** das Klebeband beidseitig klebend ist.

10. Analytisches Testelement gemäß Anspruch 9, **dadurch gekennzeichnet, daß** das Klebeband auf beiden Seiten identisch beschichtet ist.

11. Analytisches Testelement gemäß einem der Ansprüche 1 bis 10, **dadurch gekennzeichnet, daß** das analytische Testelement einen Kapillarspalt enthält.

12. Analytisches Testelement gemäß einem der Ansprüche 1 bis 11, **dadurch gekennzeichnet, daß** mindestens eine Komponente ein schichtförmiges Material ist.

## Claims

1. Analytical test element for examining a liquid sample containing a carrier and a component,
wherein the one component is selected from the group comprising a
detection layer, a separation layer, a liquid transport layer, a spreading layer and a layer for holding excess sample,
wherein the carrier is joined to the one component with the aid of an adhesive and/or the one component is joined with the aid of an adhesive to at least one other component selected from the group comprising a detection layer, a separation layer, a liquid transport layer, a spreading layer and a layer for holding excess sample,
**characterized in that**
the adhesive contains a porous solid as an adsorptive material and the porous solid is selected from the group comprising active charcoal, carbon black, aluminium oxide, silica gel or zeolite.

2. Analytical test element as claimed in claim 1, **characterized in that** the solid is not soluble in the adhesive.

3. Analytical test element as claimed in claim 1 or 2, **characterized in that** the solid is active charcoal or carbon black.

4. Analytical test element as claimed in one of the claims 1 to 3, **characterized in that** the content of the adsorptive material is 1 to 40 % by weight based on the dried total mass of the adhesive.

5. Analytical test element as claimed in one of the claims 1 to 4, **characterized in that** the adhesive sets physically.

6. Analytical test element as claimed in claim 5, **characterized in that** the adhesive is solvent-free.

7. Analytical test element as claimed in claim 6, **characterized in that** the adhesive is an acrylate adhesive.

8. Analytical test element as claimed in one of the claims 5-7, **characterized in that** the adhesive is present in the form of an adhesive tape.

9. Analytical test element as claimed in claim 8, **characterized in that** the adhesive tape is adherent on both sides.

10. Analytical test element as claimed in claim 9, **characterized in that** both sides of the adhesive tape are coated identically.

11. Analytical test element as claimed in one of the claims 1 to 10, **characterized in that** the analytical test element contains a capillary gap.

12. Analytical test element as claimed in one of the claims 1 to 11, **characterized in that** at least one component is a layered material.

## Revendications

1. Élément d'essai analytique pour l'analyse d'un échantillon liquide,
contenant un support et un composant,
dans lequel le composant est choisi parmi le groupe comprenant une couche de décèlement, une couche de séparation, une couche de transport de liquide, une couche d'étalement et une couche pour la réception de l'échantillon en excès ;
dans lequel le support est relié audit un composant à l'aide d'un adhésif, et/ou le composant est relié à l'aide d'un adhésif à au moins un composant supplémentaire choisi parmi le groupe comprenant une couche de décèlement, une couche de séparation, une couche de transport de liquide, une couche d'étalement et une couche pour la réception de l'échantillon en excès,
**caractérisé en ce que**
l'adhésif contient une substance solide poreuse à titre de matière manifestant une aptitude à l'adsorption,
la substance solide poreuse étant choisie parmi le groupe comprenant du charbon actif, du noir de carbone, de l'oxyde d'aluminium, du gel de silice ou de la zéolithe.

2. Élément d'essai analytique selon la revendication 1, **caractérisé en ce que** la substance solide n'est pas soluble dans l'adhésif.

3. Élément d'essai analytique selon la revendication 1 ou 2, **caractérisé en ce que** la substance solide est du charbon actif ou du noir de carbone.

4. Élément d'essai analytique selon l'une quelconque des revendications 1 à 3, **caractérisé en ce que** la fraction de la matière manifestant une aptitude à l'adsorption représente de 1 à 40 % en poids, rapportés à la masse totale séchée de l'adhésif.

5. Élément d'essai analytique selon l'une quelconque des revendications 1 à 4, **caractérisé en ce que** l'adhésif se durcit par voie physique.

6. Élément d'essai analytique selon la revendication 5, **caractérisé en ce que** l'adhésif est exempt de solvant.

7. Élément d'essai analytique selon la revendication 6, **caractérisé en ce que** l'adhésif est un adhésif à base d'acrylate.

8. Élément d'essai analytique selon l'une quelconque des revendications 5 à 7, **caractérisé en ce que** l'adhésif est présent sous la forme d'une bande adhésive.

9. Élément d'essai analytique selon la revendication 8, **caractérisé en ce que** la bande adhésive est une bande adhésive double face.

10. Élément d'essai analytique selon la revendication 9, **caractérisé en ce que** la bande adhésive possède un revêtement identique sur les deux faces.

11. Élément d'essai analytique selon l'une quelconque des revendications 1 à 10, **caractérisé en ce que** l'élément d'essai analytique contient une fente capillaire.

12. Élément d'essai analytique selon l'une quelconque des revendications 1 à 11, **caractérisé en ce que** au moins un composant est une matière en forme de couche.
